# EUROPEAN PATENT SPECIFICATION

(11) **EP 4 395 771 B1**
(45) Date of publication and mention of the grant of the patent: **19.11.2025**
(21) Application number: 22823616.2
(22) Date of filing: 30.08.2022
(51) Int. Cl.: A61K 31/37, A61K 31/122, A61K 31/375, A61K 31/7084, A61K 31/706, A61K 33/30, A61P 31/14, A61P 31/12, A61P 21/00

(54) **TREATING LONG COVID-19 WITH UROLITHINS**
BEHANDLUNG VON LANGEM COVID-19 MIT UROLITHINEN
TRAITEMENT DE LA COVID-19 LONGUE AU MOYEN D'UROLITHINES

(30) Priority: 30.08.2021 US 202163238659 P
(43) Date of publication of application: 10.07.2024
(73) Proprietor: Amazentis SA, 1015 Lausanne (CH)
(72) Inventor: RINSCH, Christopher, L., 1110 Morges (CH); SINGH, Anurag, 1024 Crissier (CH); D'AMICO, Davide, 1020 Renens (CH)
(74) Representative: Abel & Imray LLP
(86) International application number: PCT/IB2022/000597
(87) International publication number: WO 2023/031673

(56) References cited:
- US-A1- 2016 213 641
- WOOD EMILY ET AL: "Role of mitochondria, oxidative stress and the response to antioxidants in myalgic encephalomyelitis/chronic fatigue syndrome: A possible approach to SARS-CoV-2 'long-haulers'?", CHRONIC DISEASES AND TRANSLATIONAL MEDICINE, vol. 7, no. 1, 1 March 2021 (2021-03-01), pages 14 - 26, XP093015758, ISSN: 2589-0514, Retrieved from the Internet <URL:https://dul.usage.elsevier.com/doi/> DOI: 10.1016/j.cdtm.2020.11.002
- D'AMICO DAVIDE ET AL: "Impact of the Natural Compound Urolithin A on Health, Disease, and Aging", TRENDS IN MOLECULAR MEDICINE, ELSEVIER CURRENT TRENDS, GB, vol. 27, no. 7, 21 May 2021 (2021-05-21), pages 687 - 699, XP086675843, ISSN: 1471-4914, [retrieved on 20210521], DOI: 10.1016/J.MOLMED.2021.04.009

## Description

### RELATED APPLICATION

This application claims the benefit of priority to U.S. Provisional Patent Application No. 63/238,659, filed August 30, 2021.

### BACKGROUND

Urolithins have potent effects on the improvement of a number of health conditions, and they have been shown to be highly biologically active *in vitro* and in *vivo.* Urolithins have been proposed as treatments of a variety of conditions including conditions related to inadequate mitochondrial activity, including obesity, memory decline, reduced metabolic rate, metabolic syndrome, diabetes mellitus, cardiovascular disease, hyperlipidemia, neurodegenerative diseases, cognitive disorder, mood disorder, stress, anxiety disorder, fatty liver disease (for example, NAFLD or NASH) and for improving liver function and weight management. In particular, urolithins have been shown to have beneficial effects in the enhancement of muscle function.

The role that mitochondria, oxidative stress and antioxidants may play in the understanding of the pathophysiology and treatment of chronic fatigue has been reviewed by Wood et al., ( see Chronic diseases and Translational Medicine 7 (2021): 14-16).

### SUMMARY

The present invention is defined by the claims. Any subject-matter falling outside the scope of the claims is provided for information only.

One aspect of the invention provides a composition for use in a method of treating a subject afflicted with long COVID-19, the method comprising administering to the subject a therapeutically effective amount of an urolithin.

Another aspect of the invention provides a composition for use in a method of treating a subject afflicted with myalgic encephalomyelitis/chronic fatigue syndrome, the method comprising administering to the subject a therapeutically effective amount of an urolithin.

Methods of treatment of the human body by therapy, referred to in this description, are not part of the present invention as such, but are described here in relation to compounds and pharmaceutical compositions for use in said methods for treatment of the human body by therapy, according to the present invention.

### BRIEF DESCRIPTION OF THE DRAWING

**FIG. 1****:** Human study targeting improvement in mitochondrial function/metabolic fitness of immune cells.

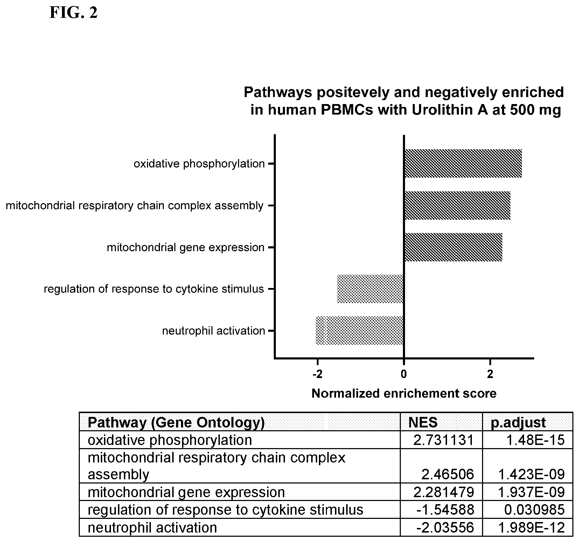

**FIG. 2****:** Pathways selectively up- or down-regulated by Urolithin A (500 mg/day) after the 28 days administration period compared to baseline (top); Normalized enriched scores ("NES") and adjusted p values ("p. adjust") for the indicated pathways. Negative NES (red - bottom two bars) indicates pathways down-regulated by Urolithin A; positive NES (blue - top three bars) indicates pathways up-regulated by Urolithin A.

### DETAILED DESCRIPTION

### Definitions

As used herein, "post-viral illness" or "post-viral syndrome" refers to a range of conditions involving physical, cognitive, emotional, and/or neurological difficulties which lingers after a person has fought off a viral infection. The illness or syndrome is triggered by a reaction to a virus, e.g., coronavirus, flu, pneumonia, or Epstein-Barr virus. The illness or syndrome may be, but is not limited to, long COVID-19, chronic fatigue syndrome, or myalgic encephalomyelitis/chronic fatigue syndrome.

As used herein, "COVID-19" refers to coronavirus disease caused by severe acute respiratory syndrome coronavirus 2 (SARS-CoV-2).

As used herein, "long COVID-19" refers to a condition wherein a subject continues to experience COVID-19 symptoms for longer than usual after initially contracting the SARS-CoV-2 virus. COVID-19 symptoms include, but are not limited to, fatigue, brain fog, headaches, dizziness, and/or shortness of breath

Long COVID is also known as post-COVID-19 syndrome, post-acute sequelae of COVID-19 (PASC), chronic COVID syndrome (CCS), and long-haul COVID.

As used herein, "myalgic encephalomyelitis/chronic fatigue syndrome" refers to a disorder most recognizably characterized by extreme tiredness, regardless of bed rest. Symptoms also include, but are not limited to, sensitivity to light, headache, tender lymph nodes, fatigue, weakness, muscle and joint pain, inability to concentrate, insomnia, forgetfulness, mood swings, confusion, low-grade fever, and depression

For convenience, before further description of the present invention, certain terms employed in the specification, examples and appended claims are collected here. These definitions should be read in light of the remainder of the disclosure and understood as by a person of skill in the art. Unless defined otherwise, all technical and scientific terms used herein have the same meaning as commonly understood by a person of ordinary skill in the art.

In order for the present invention to be more readily understood, certain terms and phrases are defined below and throughout the specification.

The articles "a" and "an" are used herein to refer to one or to more than one (i.e., to at least one) of the grammatical object of the article. By way of example, "an element" means one element or more than one element.

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified. Thus, as a non-limiting example, a reference to "A and/or B", when used in conjunction with open-ended language such as "comprising" can refer, in one embodiment, to A only (optionally including elements other than B); in another embodiment, to B only (optionally including elements other than A); in yet another embodiment, to both A and B (optionally including other elements); etc.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of" or "exactly one of," or, when used in the claims, "consisting of," will refer to the inclusion of exactly one element of a number or list of elements. In general, the term "or" as used herein shall only be interpreted as indicating exclusive alternatives (i.e., "one or the other but not both") when preceded by terms of exclusivity, such as "either," "one of," "only one of," or "exactly one of." "Consisting essentially of," when used in the claims, shall have its ordinary meaning as used in the field of patent law.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, as a non-limiting example, "at least one of A and B" (or, equivalently, "at least one of A or B," or, equivalently "at least one of A and/or B") can refer, in one embodiment, to at least one, optionally including more than one, A, with no B present (and optionally including elements other than B); in another embodiment, to at least one, optionally including more than one, B, with no A present (and optionally including elements other than A); in yet another embodiment, to at least one, optionally including more than one, A, and at least one, optionally including more than one, B (and optionally including other elements); etc.

It should also be understood that, unless clearly indicated to the contrary, in any methods claimed herein that include more than one step or act, the order of the steps or acts of the method is not necessarily limited to the order in which the steps or acts of the method are recited.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of" and "consisting essentially of" shall be closed or semi-closed transitional phrases, respectively, as set forth in the United States Patent Office Manual of Patent Examining Procedures, Section 2111.03.

Certain compounds contained in compositions of the present invention may exist in particular geometric or stereoisomeric forms. In addition, polymers of the present invention may also be optically active. The present invention contemplates all such compounds, including cis- and trans-isomers, R- and S-enantiomers, diastereomers, (D)-isomers, (L)-isomers, the racemic mixtures thereof, and other mixtures thereof, as falling within the scope of the invention. Additional asymmetric carbon atoms may be present in a substituent such as an alkyl group. All such isomers, as well as mixtures thereof, are intended to be included in this invention.

"Geometric isomer" means isomers that differ in the orientation of substituent atoms in relationship to a carbon-carbon double bond, to a cycloalkyl ring, or to a bridged bicyclic system. Atoms (other than H) on each side of a carbon- carbon double bond may be in an E (substituents are on opposite sides of the carbon- carbon double bond) or Z (substituents are oriented on the same side) configuration. "R," "S," "S*," "R*," "E," "Z," "cis," and "trans," indicate configurations relative to the core molecule. Certain of the disclosed compounds may exist in "atropisomeric" forms or as "atropisomers." Atropisomers are stereoisomers resulting from hindered rotation about single bonds where the steric strain barrier to rotation is high enough to allow for the isolation of the conformers. The compounds of the invention may be prepared as individual isomers by either isomer-specific synthesis or resolved from a mixture of isomers. Conventional resolution techniques include forming the salt of a free base of each isomer of an isomeric pair using an optically active acid (followed by fractional crystallization and regeneration of the free base), forming the salt of the acid form of each isomer of an isomeric pair using an optically active amine (followed by fractional crystallization and regeneration of the free acid), forming an ester or amide of each of the isomers of an isomeric pair using an optically pure acid, amine or alcohol (followed by chromatographic separation and removal of the chiral auxiliary), or resolving an isomeric mixture of either a starting material or a final product using various well known chromatographic methods.

If, for instance, a particular enantiomer of compound of the present invention is desired, it may be prepared by asymmetric synthesis, or by derivation with a chiral auxiliary, where the resulting diastereomeric mixture is separated and the auxiliary group cleaved to provide the pure desired enantiomers. Alternatively, where the molecule contains a basic functional group, such as amino, or an acidic functional group, such as carboxyl, diastereomeric salts are formed with an appropriate optically-active acid or base, followed by resolution of the diastereomers thus formed by fractional crystallization or chromatographic means well known in the art, and subsequent recovery of the pure enantiomers.

Percent purity by mole fraction is the ratio of the moles of the enantiomer (or diastereomer) or over the moles of the enantiomer (or diastereomer) plus the moles of its optical isomer. When the stereochemistry of a disclosed compound is named or depicted by structure, the named or depicted stereoisomer is at least about 60%, about 70%, about 80%, about 90%, about 99% or about 99.9% by mole fraction pure relative to the other stereoisomers. When a single enantiomer is named or depicted by structure, the depicted or named enantiomer is at least about 60%, about 70%, about 80%, about 90%, about 99% or about 99.9% by mole fraction pure. When a single diastereomer is named or depicted by structure, the depicted or named diastereomer is at least about 60%, about 70%, about 80%, about 90%, about 99% or about 99.9% by mole fraction pure.

When a disclosed compound is named or depicted by structure without indicating the stereochemistry, and the compound has at least one chiral center, it is to be understood that the name or structure encompasses either enantiomer of the compound free from the corresponding optical isomer, a racemic mixture of the compound or mixtures enriched in one enantiomer relative to its corresponding optical isomer. When a disclosed compound is named or depicted by structure without indicating the stereochemistry and has two or more chiral centers, it is to be understood that the name or structure encompasses a diastereomer free of other diastereomers, a number of diastereomers free from other diastereomeric pairs, mixtures of diastereomers, mixtures of diastereomeric pairs, mixtures of diastereomers in which one diastereomer is enriched relative to the other diastereomer(s) or mixtures of diastereomers in which one or more diastereomer is enriched relative to the other diastereomers. The invention embraces all of these forms.

Structures depicted herein are also meant to include compounds that differ only in the presence of one or more isotopically enriched atoms. For example, compounds produced by the replacement of a hydrogen with deuterium or tritium, or of a carbon with a ¹³C- or ¹⁴C-enriched carbon are within the scope of this invention.

The term "prodrug" as used herein encompasses compounds that, under physiological conditions, are converted into therapeutically active agents. A common method for making a prodrug is to include selected moieties that are hydrolyzed under physiological conditions to reveal the desired molecule. **In** other embodiments, the prodrug is converted by an enzymatic activity of the host animal.

The phrase "pharmaceutically acceptable excipient" or "pharmaceutically acceptable carrier" as used herein means a pharmaceutically acceptable material, composition or vehicle, such as a liquid or solid filler, diluent, excipient, solvent or encapsulating material, involved in carrying or transporting the subject chemical from one organ or portion of the body, to another organ or portion of the body. Each carrier must be "acceptable" in the sense of being compatible with the other ingredients of the formulation, not injurious to the patient, and substantially non-pyrogenic. Some examples of materials which can serve as pharmaceutically acceptable carriers include: (1) sugars, such as lactose, glucose, and sucrose; (2) starches, such as corn starch and potato starch; (3) cellulose, and its derivatives, such as sodium carboxymethyl cellulose, ethyl cellulose, and cellulose acetate; (4) powdered tragacanth; (5) malt; (6) gelatin; (7) talc; (8) excipients, such as cocoa butter and suppository waxes; (9) oils, such as peanut oil, cottonseed oil, safflower oil, sesame oil, olive oil, corn oil, and soybean oil; (10) glycols, such as propylene glycol; (11) polyols, such as glycerin, sorbitol, mannitol, and polyethylene glycol; (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) phosphate buffer solutions; and (21) other non-toxic compatible substances employed in pharmaceutical formulations. **In** certain embodiments, pharmaceutical compositions of the present invention are non-pyrogenic, i.e., do not induce significant temperature elevations when administered to a patient.

The term "pharmaceutically acceptable salts" refers to the relatively non-toxic, inorganic and organic acid addition salts of the compound(s). These salts can be prepared in situ during the final isolation and purification of the compound(s), or by separately reacting a purified compound(s) in its free base form with a suitable organic or inorganic acid, and isolating the salt thus formed. Representative salts include the hydrobromide, hydrochloride, sulfate, bisulfate, phosphate, nitrate, acetate, valerate, oleate, palmitate, stearate, laurate, benzoate, lactate, phosphate, tosylate, citrate, maleate, fumarate, succinate, tartrate, naphthylate, mesylate, glucoheptonate, lactobionate, and laurylsulphonate salts, and the like. (See, for example, Berge et al. (1977) "Pharmaceutical Salts", J. Pharm. Sci. 66:1-19.)

In other cases, the compounds useful in the methods of the present invention may contain one or more acidic functional groups and, thus, are capable of forming pharmaceutically acceptable salts with pharmaceutically acceptable bases. The term "pharmaceutically acceptable salts" in these instances refers to the relatively non-toxic inorganic and organic base addition salts of a compound(s). These salts can likewise be prepared in situ during the final isolation and purification of the compound(s), or by separately reacting the purified compound(s) in its free acid form with a suitable base, such as the hydroxide, carbonate, or bicarbonate of a pharmaceutically acceptable metal cation, with ammonia, or with a pharmaceutically acceptable organic primary, secondary, or tertiary amine. Representative alkali or alkaline earth salts include the lithium, sodium, potassium, calcium, magnesium, and aluminum salts, and the like. Representative organic amines useful for the formation of base addition salts include ethylamine, diethylamine, ethylenediamine, ethanolamine, diethanolamine, piperazine, and the like (see, for example, Berge et al., *supra*).

The term "pharmaceutically acceptable cocrystals" refers to solid coformers that do not form formal ionic interactions with the small molecule.

A "therapeutically effective amount" (or "effective amount") of a compound with respect to use in treatment, refers to an amount of the compound in a preparation which, when administered as part of a desired dosage regimen (to a mammal, preferably a human) alleviates a symptom, ameliorates a condition, or slows the onset of disease conditions according to clinically acceptable standards for the disorder or condition to be treated or the cosmetic purpose, e.g., at a reasonable benefit/risk ratio applicable to any medical treatment.

The term "prophylactic or therapeutic" treatment is art-recognized and includes administration to the host of one or more of the subject compositions. If it is administered prior to clinical manifestation of the unwanted condition (e.g., disease or other unwanted state of the host animal) then the treatment is prophylactic, (i.e., it protects the host against developing the unwanted condition), whereas if it is administered after manifestation of the unwanted condition, the treatment is therapeutic, (i.e., it is intended to diminish, ameliorate, or stabilize the existing unwanted condition or side effects thereof).

The term "patient" or "subject" refers to a mammal in need of a particular treatment. In certain embodiments, a patient is a primate, canine, feline, or equine. In certain embodiments, a patient is a human.

As used herein, "small molecules" refers to small organic or inorganic molecules of molecular weight below about 3,000 Daltons. In general, small molecules useful for the invention have a molecular weight of less than 3,000 Daltons (Da). The small molecules can be, e.g., from at least about 100 Da to about 3,000 Da (e.g., between about 100 to about 3,000 Da, about 100 to about 2500 Da, about 100 to about 2,000 Da, about 100 to about 1,750 Da, about 100 to about 1,500 Da, about 100 to about 1,250 Da, about 100 to about 1,000 Da, about 100 to about 750 Da, about 100 to about 500 Da, about 200 to about 1500, about 500 to about 1000, about 300 to about 1000 Da, or about 100 to about 250 Da).

In some embodiments, a "small molecule" refers to an organic, inorganic, or organometallic compound typically having a molecular weight of less than about 1000. In some embodiments, a small molecule is an organic compound, with a size on the order of 1 nm. In some embodiments, small molecule drugs of the invention encompass oligopeptides and other biomolecules having a molecular weight of less than about 1000.

An "effective amount" is an amount sufficient to effect beneficial or desired results. For example, a therapeutic amount is one that achieves the desired therapeutic effect. This amount can be the same or different from a prophylactically effective amount, which is an amount necessary to prevent onset of disease or disease symptoms. An effective amount can be administered in one or more administrations, applications or dosages. A therapeutically effective amount of a composition depends on the composition selected. The compositions can be administered from one or more times per day to one or more times per week; including once every other day. The skilled artisan will appreciate that certain factors may influence the dosage and timing required to effectively treat a subject, including but not limited to the severity of the disease or disorder, previous treatments, the general health and/or age of the subject, and other diseases present. Moreover, treatment of a subject with a therapeutically effective amount of the compositions described herein can include a single treatment or a series of treatments.

The terms "decrease," "reduce," "reduced", "reduction", "decrease," and "inhibit" are all used herein generally to mean a decrease by a statistically significant amount relative to a reference. However, for avoidance of doubt, "reduce," "reduction" or "decrease" or "inhibit" typically means a decrease by at least 10% as compared to a reference level and can include, for example, a decrease by at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 98%, at least about 99%, up to and including, for example, the complete absence of the given entity or parameter as compared to the reference level, or any decrease between 10-99% as compared to the absence of a given treatment.

The terms "increased", "increase" or "enhance" or "activate" are all used herein to generally mean an increase by a statically significant amount; for the avoidance of any doubt, the terms "increased", "increase" or "enhance" or "activate" means an increase of at least 10% as compared to a reference level, for example an increase of at least about 20%, or at least about 30%, or at least about 40%, or at least about 50%, or at least about 60%, or at least about 70%, or at least about 80%, or at least about 90% or up to and including a 100% increase or any increase between 10-100% as compared to a reference level, or at least about a 2-fold, or at least about a 3-fold, or at least about a 4-fold, or at least about a 5-fold or at least about a 10-fold increase, or any increase between 2-fold and 10-fold or greater as compared to a reference level.

As used herein, the term "modulate" includes up-regulation and down-regulation, e.g., enhancing or inhibiting a response.

A "radiopharmaceutical agent," as defined herein, refers to a pharmaceutical agent which contains at least one radiation-emitting radioisotope. Radiopharmaceutical agents are routinely used in nuclear medicine for the diagnosis and/or therapy of various diseases. The radiolabelled pharmaceutical agent, for example, a radiolabelled antibody, contains a radioisotope (RI) which serves as the radiation source. As contemplated herein, the term "radioisotope" includes metallic and non-metallic radioisotopes. The radioisotope is chosen based on the medical application of the radiolabeled pharmaceutical agents. When the radioisotope is a metallic radioisotope, a chelator is typically employed to bind the metallic radioisotope to the rest of the molecule. When the radioisotope is a non-metallic radioisotope, the non-metallic radioisotope is typically linked directly, or via a linker, to the rest of the molecule.

For purposes of this invention, the chemical elements are identified in accordance with the Periodic Table of the Elements, CAS version, Handbook of Chemistry and Physics, 67th Ed., 1986-87, inside cover.

### Methods of Treatment

One aspect of the invention relates to a method of treating a subject afflicted with symptoms associated with recovery from COVID-19, comprising administering to the subject a therapeutically effective amount of an urolithin.

In certain embodiments, the urolithin treats symptoms associated with recovery from COVID-19.

In certain embodiments, the symptoms comprise post-exertional malaise (PEM), problems with memory or concentration, sore throat, headache, muscle or joint pain, dizziness, brain fog, and/or unrefreshing sleep.

In certain embodiments, the symptoms comprise fatigue, brain fog, headaches, dizziness, and/or shortness of breath.

In certain embodiments, the symptoms comprise fatigue.

In certain embodiments, the urolithin treats a secondary disease or disorder associated with recovery from COVID-19 in the subject.

In certain embodiments, the urolithin treats a secondary disease or disorder triggered by COVID-19 in the subject.

In certain embodiments, the secondary disease or disorder is myalgic encephalomyelitis/chronic fatigue syndrome.

In certain embodiments, the long COVID-19 is caused by an infection with a delta variant of SARS-CoV-2 in the subject.

In certain embodiments, the long COVID-19 is caused by an infection with an omicron variant of SARS-CoV-2 in the subject.

Another aspect of the invention relates to a method of treating a subject afflicted with myalgic encephalomyelitis/chronic fatigue syndrome, comprising administering to the subject a therapeutically effective amount of an urolithin.

In certain embodiments, the compound treats symptoms associated with myalgic encephalomyelitis/chronic fatigue syndrome.

In certain embodiments, the symptoms comprise fatigue, post-exertional malaise (PEM) Problems with memory or concentration, sore throat, headache, muscle or joint pain, dizziness, or unrefreshing sleep.

In certain embodiments, the myalgic encephalomyelitis/chronic fatigue syndrome is triggered by COVID-19 in the subject.

In certain embodiments, the myalgic encephalomyelitis/chronic fatigue syndrome is triggered by long COVID-19 in the subject.

In certain embodiments, the subject was previously afflicted with COVID-19 prior to the onset of myalgic encephalomyelitis/chronic fatigue syndrome.

In certain embodiments, the subject was previously afflicted with long COVID-19 prior to the onset of myalgic encephalomyelitis/chronic fatigue syndrome.In certain embodiments, the urolithin is urolithin A (UA). Urolithin A has the structure:

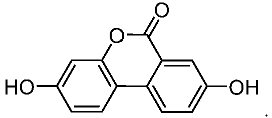

In certain embodiments, the urolithin is urolithin B (UB). Urolithin B has the structure:

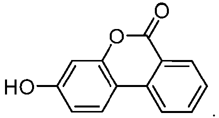

In certain embodiments, the urolithin is urolithin C (UC). Urolithin C has the structure:

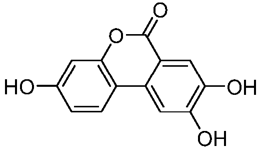

In certain embodiments, the urolithin is urolithin D (UD). Urolithin D has the structure:

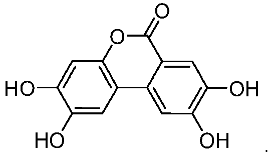

In certain embodiments, the subject is a human.

In certain embodiments, the subject is a child.

In certain embodiments, the subject is a juvenile.

In certain embodiments, the subject is an adult.

In certain embodiments, the subject is elderly.

In certain embodiments, the urolithin is orally administered to the subject.

In certain embodiments, the urolithin is intravenously administered to the subject.In certain embodiments, the subject is concurrently administered a nutrient.

In certain embodiments, the nutrient is vitamin C or zinc.

In certain embodiments, the subject is concurrently administered a coenzyme.

In certain embodiments, the coenzyme is coenzyme Q10 (CoQ10) or nicotinamide adenine dinucleotide (NAD+).

In certain embodiments, the subject is concurrently administered a nicotinamide adenine dinucleotide (NAD+) precursor.

In certain embodiments, the precursor is nicotinamide riboside.

### Compositions, Routes of Administration, and Dosing

In certain embodiments, a pharmaceutical composition of the invention further comprises at least one additional pharmaceutically active agent other than a urolithin of the invention.

In certain embodiments, the urolithin is present in a pharmaceutical composition further comprising a nutrient.

In certain embodiments, the nutrient is vitamin C or zinc.

In certain embodiments, the urolithin is present in a pharmaceutical composition further comprising a coenzyme or a nicotinamide adenine dinucleotide (NAD+) precursor.

In certain embodiments, the coenzyme is coenzyme Q10 (CoQ10) or nicotinamide adenine dinucleotide (NAD+).

In certain embodiments, the precursor is nicotinamide riboside.

In certain embodiments, the pharmaceutical composition further comprises a pharmaceutically acceptable carrier.

In certain embodiments, the pharmaceutical composition comprises a plurality of compounds of the invention; and a pharmaceutically acceptable carrier.

As stated above, an "effective amount" refers to any amount that is sufficient to achieve a desired biological effect. Combined with the teachings provided herein, by choosing among the various active compounds and weighing factors such as potency, relative bioavailability, patient body weight, severity of adverse side-effects and mode of administration, an effective prophylactic or therapeutic treatment regimen can be planned which does not cause substantial unwanted toxicity and yet is effective to treat the particular subject. The effective amount for any particular application can vary depending on such factors as the disease or condition being treated, the particular compound of the invention being administered, the size of the subject, or the severity of the disease or condition. One of ordinary skill in the art can empirically determine the effective amount of a particular compound of the invention and/or other therapeutic agent without necessitating undue experimentation. A maximum dose may be used, that is, the highest safe dose according to some medical judgment. Multiple doses per day may be contemplated to achieve appropriate systemic levels of compounds. Appropriate systemic levels can be determined by, for example, measurement of the patient's peak or sustained plasma level of the drug. "Dose" and "dosage" are used interchangeably herein.

In certain embodiments, intravenous administration of a compound may typically be from 0.1 mg/kg/day to 20 mg/kg/day. In one embodiment, intravenous administration of a compound may typically be from 0.1 mg/kg/day to 2 mg/kg/day. In one embodiment, intravenous administration of a compound may typically be from 0.5 mg/kg/day to 5 mg/kg/day. In one embodiment, intravenous administration of a compound may typically be from 1 mg/kg/day to 20 mg/kg/day. In one embodiment, intravenous administration of a compound may typically be from 1 mg/kg/day to 10 mg/kg/day.

Generally, daily oral doses of a compound will be, for human subjects, from about 0.01 milligrams/kg per day to 1000 milligrams/kg per day. It is expected that oral doses in the range of 0.5 to 50 milligrams/kg, in one or more administrations per day, will yield therapeutic results. Dosage may be adjusted appropriately to achieve desired drug levels, local or systemic, depending upon the mode of administration. For example, it is expected that intravenous administration would be from one order to several orders of magnitude lower dose per day. In the event that the response in a subject is insufficient at such doses, even higher doses (or effective higher doses by a different, more localized delivery route) may be employed to the extent that patient tolerance permits. Multiple doses per day are contemplated to achieve appropriate systemic levels of the compound.

For any compound described herein the therapeutically effective amount can be initially determined from animal models. A therapeutically effective dose can also be determined from human data for compounds which have been tested in humans and for compounds which are known to exhibit similar pharmacological activities, such as other related active agents. Higher doses may be required for parenteral administration. The applied dose can be adjusted based on the relative bioavailability and potency of the administered compound. Adjusting the dose to achieve maximal efficacy based on the methods described above and other methods as are well-known in the art is well within the capabilities of the ordinarily skilled artisan.

The formulations of the invention can be administered in pharmaceutically acceptable solutions, which may routinely contain pharmaceutically acceptable concentrations of salt, buffering agents, preservatives, compatible carriers, adjuvants, and optionally other therapeutic ingredients.

For use in therapy, an effective amount of the compound can be administered to a subject by any mode that delivers the compound to the desired surface. Administering a pharmaceutical composition may be accomplished by any means known to the skilled artisan. Routes of administration include but are not limited to intravenous, intramuscular, intraperitoneal, intravesical (urinary bladder), oral, subcutaneous, direct injection (for example, into a tumor or abscess), mucosal (e.g., topical to eye), inhalation, and topical.

For intravenous and other parenteral routes of administration, a compound of the invention can be formulated as a lyophilized preparation, as a lyophilized preparation of liposome-intercalated or -encapsulated active compound, as a lipid complex in aqueous suspension, or as a salt complex. Lyophilized formulations are generally reconstituted in suitable aqueous solution, e.g., in sterile water or saline, shortly prior to administration.

For oral administration, the compounds can be formulated readily by combining the active compound(s) with pharmaceutically acceptable carriers well known in the art. Such carriers enable the compounds of the invention to be formulated as tablets, pills, dragees, capsules, liquids, gels, syrups, slurries, suspensions and the like, for oral ingestion by a subject to be treated. Pharmaceutical preparations for oral use can be obtained as solid excipient, optionally grinding a resulting mixture, and processing the mixture of granules, after adding suitable auxiliaries, if desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethyl-cellulose, sodium carboxymethylcellulose, and/or polyvinylpyrrolidone (PVP). If desired, disintegrating agents may be added, such as the cross-linked polyvinyl pyrrolidone, agar, or alginic acid or a salt thereof such as sodium alginate. Optionally the oral formulations may also be formulated in saline or buffers, e.g., EDTA for neutralizing internal acid conditions or may be administered without any carriers.

Also specifically contemplated are oral dosage forms of the above component or components. The component or components may be chemically modified so that oral delivery of the derivative is efficacious. Generally, the chemical modification contemplated is the attachment of at least one moiety to the component molecule itself, where said moiety permits (a) inhibition of acid hydrolysis; and (b) uptake into the blood stream from the stomach or intestine. Also desired is the increase in overall stability of the component or components and increase in circulation time in the body. Examples of such moieties include: polyethylene glycol, copolymers of ethylene glycol and propylene glycol, carboxymethyl cellulose, dextran, polyvinyl alcohol, polyvinyl pyrrolidone and polyproline. Abuchowski and Davis, "Soluble Polymer-Enzyme Adducts", In: Enzymes as Drugs, Hocenberg and Roberts, eds., Wiley-Interscience, New York, N.Y., pp. 367-383 (1981); Newmark et al., J Appl Biochem 4:185-9 (1982). Other polymers that could be used are poly-1,3-dioxolane and poly-1,3,6-tioxocane. For pharmaceutical usage, as indicated above, polyethylene glycol moieties are suitable.

For the component (or derivative) the location of release may be the stomach, the small intestine (the duodenum, the jejunum, or the ileum), or the large intestine. One skilled in the art has available formulations which will not dissolve in the stomach, yet will release the material in the duodenum or elsewhere in the intestine. Preferably, the release will avoid the deleterious effects of the stomach environment, either by protection of the compound of the invention (or derivative) or by release of the biologically active material beyond the stomach environment, such as in the intestine.

To ensure full gastric resistance a coating impermeable to at least pH 5.0 is essential. Examples of the more common inert ingredients that are used as enteric coatings are cellulose acetate trimellitate (CAT), hydroxypropylmethylcellulose phthalate (HPMCP), HPMCP 50, HPMCP 55, polyvinyl acetate phthalate (PVAP), Eudragit L30D, Aquateric, cellulose acetate phthalate (CAP), Eudragit L, Eudragit S, and shellac. These coatings may be used as mixed films.

A coating or mixture of coatings can also be used on tablets, which are not intended for protection against the stomach. This can include sugar coatings, or coatings which make the tablet easier to swallow. Capsules may consist of a hard shell (such as gelatin) for delivery of dry therapeutic (e.g., powder); for liquid forms, a soft gelatin shell may be used. The shell material of cachets could be thick starch or other edible paper. For pills, lozenges, molded tablets or tablet triturates, moist massing techniques can be used.

The therapeutic can be included in the formulation as fine multi-particulates in the form of granules or pellets of particle size about 1 mm. The formulation of the material for capsule administration could also be as a powder, lightly compressed plugs or even as tablets. The therapeutic could be prepared by compression.

Colorants and flavoring agents may all be included. For example, the compound of the invention (or derivative) may be formulated (such as by liposome or microsphere encapsulation) and then further contained within an edible product, such as a refrigerated beverage containing colorants and flavoring agents.

One may dilute or increase the volume of the therapeutic with an inert material. These diluents could include carbohydrates, especially mannitol, α-lactose, anhydrous lactose, cellulose, sucrose, modified dextrans and starch. Certain inorganic salts may be also be used as fillers including calcium triphosphate, magnesium carbonate and sodium chloride. Some commercially available diluents are Fast-Flo, Emdex, STA-Rx 1500, Emcompress and Avicell.

Disintegrants may be included in the formulation of the therapeutic into a solid dosage form. Materials used as disintegrates include but are not limited to starch, including the commercial disintegrant based on starch, Explotab. Sodium starch glycolate, Amberlite, sodium carboxymethylcellulose, ultramylopectin, sodium alginate, gelatin, orange peel, acid carboxymethyl cellulose, natural sponge and bentonite may all be used. Another form of the disintegrants are the insoluble cationic exchange resins. Powdered gums may be used as disintegrants and as binders and these can include powdered gums such as agar, Karaya or tragacanth. Alginic acid and its sodium salt are also useful as disintegrants.

Binders may be used to hold the therapeutic agent together to form a hard tablet and include materials from natural products such as acacia, tragacanth, starch and gelatin. Others include methyl cellulose (MC), ethyl cellulose (EC) and carboxymethyl cellulose (CMC). Polyvinyl pyrrolidone (PVP) and hydroxypropylmethyl cellulose (HPMC) could both be used in alcoholic solutions to granulate the therapeutic.

An anti-frictional agent may be included in the formulation of the therapeutic to prevent sticking during the formulation process. Lubricants may be used as a layer between the therapeutic and the die wall, and these can include but are not limited to; stearic acid including its magnesium and calcium salts, polytetrafluoroethylene (PTFE), liquid paraffin, vegetable oils and waxes. Soluble lubricants may also be used such as sodium lauryl sulfate, magnesium lauryl sulfate, polyethylene glycol of various molecular weights, Carbowax 4000 and 6000.

Glidants that might improve the flow properties of the drug during formulation and to aid rearrangement during compression might be added. The glidants may include starch, talc, pyrogenic silica and hydrated silicoaluminate.

To aid dissolution of the therapeutic into the aqueous environment a surfactant might be added as a wetting agent. Surfactants may include anionic detergents such as sodium lauryl sulfate, dioctyl sodium sulfosuccinate and dioctyl sodium sulfonate. Cationic detergents which can be used and can include benzalkonium chloride and benzethonium chloride. Potential non-ionic detergents that could be included in the formulation as surfactants include lauromacrogol 400, polyoxyl 40 stearate, polyoxyethylene hydrogenated castor oil 10, 50 and 60, glycerol monostearate, polysorbate 40, 60, 65 and 80, sucrose fatty acid ester, methyl cellulose and carboxymethyl cellulose. These surfactants could be present in the formulation of the compound of the invention or derivative either alone or as a mixture in different ratios.

Pharmaceutical preparations which can be used orally include push-fit capsules made of gelatin, as well as soft, sealed capsules made of gelatin and a plasticizer, such as glycerol or sorbitol. The push-fit capsules can contain the active ingredients in admixture with filler such as lactose, binders such as starches, and/or lubricants such as talc or magnesium stearate and, optionally, stabilizers. In soft capsules, the active compounds may be dissolved or suspended in suitable liquids, such as fatty oils, liquid paraffin, or liquid polyethylene glycols. In addition, stabilizers may be added. Microspheres formulated for oral administration may also be used. Such microspheres have been well defined in the art. All formulations for oral administration should be in dosages suitable for such administration.

For buccal administration, the compositions may take the form of tablets or lozenges formulated in conventional manner.

For topical administration, the compound may be formulated as solutions, gels, ointments, creams, suspensions, etc. as are well-known in the art. Systemic formulations include those designed for administration by injection, e.g., subcutaneous, intravenous, intramuscular, intrathecal or intraperitoneal injection, as well as those designed for transdermal, transmucosal oral or pulmonary administration.

For administration by inhalation, compounds for use according to the present invention may be conveniently delivered in the form of an aerosol spray presentation from pressurized packs or a nebulizer, with the use of a suitable propellant, e.g., dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount. Capsules and cartridges of e.g., gelatin for use in an inhaler or insufflator may be formulated containing a powder mix of the compound and a suitable powder base such as lactose or starch.

Also contemplated herein is pulmonary delivery of the compounds disclosed herein (or salts thereof). The compound is delivered to the lungs of a mammal while inhaling and traverses across the lung epithelial lining to the blood stream. Other reports of inhaled molecules include Adjei et al., Pharm Res 7:565-569 (1990); Adjei et al., Int J Pharmaceutics 63:135-144 (1990) (leuprolide acetate); Braquet et al., J Cardiovasc Pharmacol 13(suppl. 5):143-146 (1989) (endothelin-1); Hubbard et al., Annal Int Med 3:206-212 (1989) (α1-antitrypsin); Smith et al., 1989, J Clin Invest 84:1145-1146 (a-1-proteinase); Oswein et al., 1990, "Aerosolization of Proteins", Proceedings of Symposium on Respiratory Drug Delivery II, Keystone, Colorado, March, (recombinant human growth hormone); Debs et al., 1988, J Immunol 140:3482-3488 (interferon-gamma and tumor necrosis factor alpha) and Platz et al., U.S. Pat. No. 5,284,656 (granulocyte colony stimulating factor).
A method and composition for pulmonary delivery of drugs for systemic effect is described in U.S. Pat. No. 5,451,569 issued Sep. 19, 1995 to Wong et al

Contemplated for use in the practice of this invention are a wide range of mechanical devices designed for pulmonary delivery of therapeutic products, including but not limited to nebulizers, metered dose inhalers, and powder inhalers, all of which are familiar to those skilled in the art.

Some specific examples of commercially available devices suitable for the practice of this invention are the Ultravent nebulizer, manufactured by Mallinckrodt, Inc., St. Louis, Mo.; the Acorn II nebulizer, manufactured by Marquest Medical Products, Englewood, Colo.; the Ventolin metered dose inhaler, manufactured by Glaxo Inc., Research Triangle Park, North Carolina; and the Spinhaler powder inhaler, manufactured by Fisons Corp., Bedford, Mass.

All such devices require the use of formulations suitable for the dispensing of the compounds of the invention. Typically, each formulation is specific to the type of device employed and may involve the use of an appropriate propellant material, in addition to the usual diluents, adjuvants and/or carriers useful in therapy. Also, the use of liposomes, microcapsules or microspheres, inclusion complexes, or other types of carriers is contemplated. Chemically modified compound of the invention may also be prepared in different formulations depending on the type of chemical modification or the type of device employed.

Formulations suitable for use with a nebulizer, either jet or ultrasonic, will typically comprise a compound of the invention (or derivative) dissolved in water at a concentration of about 0.1 to 25 mg of biologically active compound of the invention per mL of solution. The formulation may also include a buffer and a simple sugar (e.g., for inhibitor stabilization and regulation of osmotic pressure). The nebulizer formulation may also contain a surfactant, to reduce or prevent surface induced aggregation of the compound of the invention caused by atomization of the solution in forming the aerosol.

Formulations for use with a metered-dose inhaler device will generally comprise a finely divided powder containing the compound of the invention (or derivative) suspended in a propellant with the aid of a surfactant. The propellant may be any conventional material employed for this purpose, such as a chlorofluorocarbon, a hydrochlorofluorocarbon, a hydrofluorocarbon, or a hydrocarbon, including trichlorofluoromethane, dichlorodifluoromethane, dichlorotetrafluoroethanol, and 1,1,1,2-tetrafluoroethane, or combinations thereof. Suitable surfactants include sorbitan trioleate and soya lecithin. Oleic acid may also be useful as a surfactant.

Formulations for dispensing from a powder inhaler device will comprise a finely divided dry powder containing compound of the invention (or derivative) and may also include a bulking agent, such as lactose, sorbitol, sucrose, or mannitol in amounts which facilitate dispersal of the powder from the device, e.g., 50 to 90% by weight of the formulation. The compound of the invention (or derivative) should advantageously be prepared in particulate form with an average particle size of less than 10 micrometers (µm), most preferably 0.5 to 5 µm, for most effective delivery to the deep lung.

Nasal delivery of a pharmaceutical composition of the present invention is also contemplated. Nasal delivery allows the passage of a pharmaceutical composition of the present invention to the blood stream directly after administering the therapeutic product to the nose, without the necessity for deposition of the product in the lung. Formulations for nasal delivery include those with dextran or cyclodextran.

For nasal administration, a useful device is a small, hard bottle to which a metered dose sprayer is attached. In one embodiment, the metered dose is delivered by drawing the pharmaceutical composition of the present invention solution into a chamber of defined volume, which chamber has an aperture dimensioned to aerosolize and aerosol formulation by forming a spray when a liquid in the chamber is compressed. The chamber is compressed to administer the pharmaceutical composition of the present invention. In a specific embodiment, the chamber is a piston arrangement. Such devices are commercially available.

Alternatively, a plastic squeeze bottle with an aperture or opening dimensioned to aerosolize an aerosol formulation by forming a spray when squeezed is used. The opening is usually found in the top of the bottle, and the top is generally tapered to partially fit in the nasal passages for efficient administration of the aerosol formulation. Preferably, the nasal inhaler will provide a metered amount of the aerosol formulation, for administration of a measured dose of the drug.

The compounds, when it is desirable to deliver them systemically, may be formulated for parenteral administration by injection, e.g., by bolus injection or continuous infusion. Formulations for injection may be presented in unit dosage form, e.g., in ampoules or in multi-dose containers, with an added preservative. The compositions may take such forms as suspensions, solutions or emulsions in oily or aqueous vehicles, and may contain formulatory agents such as suspending, stabilizing and/or dispersing agents.

Pharmaceutical formulations for parenteral administration include aqueous solutions of the active compounds in water-soluble form. Additionally, suspensions of the active compounds may be prepared as appropriate oily injection suspensions. Suitable lipophilic solvents or vehicles include fatty oils such as sesame oil, or synthetic fatty acid esters, such as ethyl oleate or triglycerides, or liposomes. Aqueous injection suspensions may contain substances which increase the viscosity of the suspension, such as sodium carboxymethylcellulose, sorbitol, or dextran. Optionally, the suspension may also contain suitable stabilizers or agents which increase the solubility of the compounds to allow for the preparation of highly concentrated solutions.

Alternatively, the active compounds may be in powder form for constitution with a suitable vehicle, e.g., sterile pyrogen-free water, before use.

The compounds may also be formulated in rectal or vaginal compositions such as suppositories or retention enemas, e.g., containing conventional suppository bases such as cocoa butter or other glycerides.

In addition to the formulations described above, a compound may also be formulated as a depot preparation. Such long acting formulations may be formulated with suitable polymeric or hydrophobic materials (for example as an emulsion in an acceptable oil) or ion exchange resins, or as sparingly soluble derivatives, for example, as a sparingly soluble salt.

The pharmaceutical compositions also may comprise suitable solid or gel phase carriers or excipients. Examples of such carriers or excipients include but are not limited to calcium carbonate, calcium phosphate, various sugars, starches, cellulose derivatives, gelatin, and polymers such as polyethylene glycols.

Suitable liquid or solid pharmaceutical preparation forms are, for example, aqueous or saline solutions for inhalation, microencapsulated, encochleated, coated onto microscopic gold particles, contained in liposomes, nebulized, aerosols, pellets for implantation into the skin, or dried onto a sharp object to be scratched into the skin. The pharmaceutical compositions also include granules, powders, tablets, coated tablets, (micro)capsules, suppositories, syrups, emulsions, suspensions, creams, drops or preparations with protracted release of active compounds, in whose preparation excipients and additives and/or auxiliaries such as disintegrants, binders, coating agents, swelling agents, lubricants, flavorings, sweeteners or solubilizers are customarily used as described above. The pharmaceutical compositions are suitable for use in a variety of drug delivery systems. For a brief review of methods for drug delivery, see Langer R, Science 249:1527-33 (1990).

The compound of the invention and optionally other therapeutics may be administered *per se* (neat) or in the form of a pharmaceutically acceptable salt or cocrystal. When used in medicine the salts or cocrystals should be pharmaceutically acceptable, but nonpharmaceutically acceptable salts or cocrystals may conveniently be used to prepare pharmaceutically acceptable salts or cocrystals thereof. Such salts include, but are not limited to, those prepared from the following acids: hydrochloric, hydrobromic, sulphuric, nitric, phosphoric, maleic, acetic, salicylic, p-toluene sulphonic, tartaric, citric, methane sulphonic, formic, malonic, succinic, naphthalene-2-sulphonic, and benzene sulphonic. Also, such salts can be prepared as alkaline metal or alkaline earth salts, such as sodium, potassium or calcium salts of the carboxylic acid group.

Suitable buffering agents include: acetic acid and a salt (1-2% w/v); citric acid and a salt (1-3% w/v); boric acid and a salt (0.5-2.5% w/v); and phosphoric acid and a salt (0.8-2% w/v). Suitable preservatives include benzalkonium chloride (0.003-0.03% w/v); chlorobutanol (0.3-0.9% w/v); parabens (0.01-0.25% w/v) and thimerosal (0.004-0.02% w/v).

Pharmaceutical compositions of the invention contain an effective amount of a compound as described herein and optionally therapeutic agents included in a pharmaceutically acceptable carrier. The term "pharmaceutically acceptable carrier" means one or more compatible solid or liquid filler, diluents or encapsulating substances which are suitable for administration to a human or other vertebrate animal. The term "carrier" denotes an organic or inorganic ingredient, natural or synthetic, with which the active ingredient is combined to facilitate the application. The components of the pharmaceutical compositions also are capable of being commingled with the compounds of the present invention, and with each other, in a manner such that there is no interaction which would substantially impair the desired pharmaceutical efficiency.

The therapeutic agent(s), including specifically but not limited to a compound of the invention, may be provided in particles. Particles as used herein means nanoparticles or microparticles (or in some instances larger particles) which can consist in whole or in part of the compound of the invention or the other therapeutic agent(s) as described herein. The particles may contain the therapeutic agent(s) in a core surrounded by a coating, including, but not limited to, an enteric coating. The therapeutic agent(s) also may be dispersed throughout the particles. The therapeutic agent(s) also may be adsorbed into the particles. The particles may be of any order release kinetics, including zero-order release, first-order release, second-order release, delayed release, sustained release, immediate release, and any combination thereof, etc. The particle may include, in addition to the therapeutic agent(s), any of those materials routinely used in the art of pharmacy and medicine, including, but not limited to, erodible, nonerodible, biodegradable, or nonbiodegradable material or combinations thereof. The particles may be microcapsules which contain the compound of the invention in a solution or in a semi-solid state. The particles may be of virtually any shape.

Both non-biodegradable and biodegradable polymeric materials can be used in the manufacture of particles for delivering the therapeutic agent(s). Such polymers may be natural or synthetic polymers. The polymer is selected based on the period of time over which release is desired. Bioadhesive polymers of particular interest include bioerodible hydrogels described in Sawhney H S et al. (1993) Macromolecules 26:581-7. These include polyhyaluronic acids, casein, gelatin, glutin, polyanhydrides, polyacrylic acid, alginate, chitosan, poly(methyl methacrylates), poly(ethyl methacrylates), poly(butylmethacrylate), poly(isobutyl methacrylate), poly(hexylmethacrylate), poly(isodecyl methacrylate), poly(lauryl methacrylate), poly(phenyl methacrylate), poly(methyl acrylate), poly(isopropyl acrylate), poly(isobutyl acrylate), and poly(octadecyl acrylate).

The therapeutic agent(s) may be contained in controlled release systems. The term "controlled release" is intended to refer to any drug-containing formulation in which the manner and profile of drug release from the formulation are controlled. This refers to immediate as well as non-immediate release formulations, with non-immediate release formulations including but not limited to sustained release and delayed release formulations. The term "sustained release" (also referred to as "extended release") is used in its conventional sense to refer to a drug formulation that provides for gradual release of a drug over an extended period of time, and that preferably, although not necessarily, results in substantially constant blood levels of a drug over an extended time period. The term "delayed release" is used in its conventional sense to refer to a drug formulation in which there is a time delay between administration of the formulation and the release of the drug there from. "Delayed release" may or may not involve gradual release of drug over an extended period of time, and thus may or may not be "sustained release."

Use of a long-term sustained release implant may be particularly suitable for treatment of chronic conditions. "Long-term" release, as used herein, means that the implant is constructed and arranged to deliver therapeutic levels of the active ingredient for at least 7 days, and preferably 30-60 days. Long-term sustained release implants are well-known to those of ordinary skill in the art and include some of the release systems described above.

It will be understood by one of ordinary skill in the relevant arts that other suitable modifications and adaptations to the compositions and methods described herein are readily apparent from the description of the invention contained herein in view of information known to the ordinarily skilled artisan, and may be made without departing from the scope of the invention or any embodiment thereof. Having now described the present invention in detail, the same will be more clearly understood by reference to the following examples, which are included herewith for purposes of illustration only and are not intended to be limiting of the invention.

### Example 1. Human study of Long COVID-19

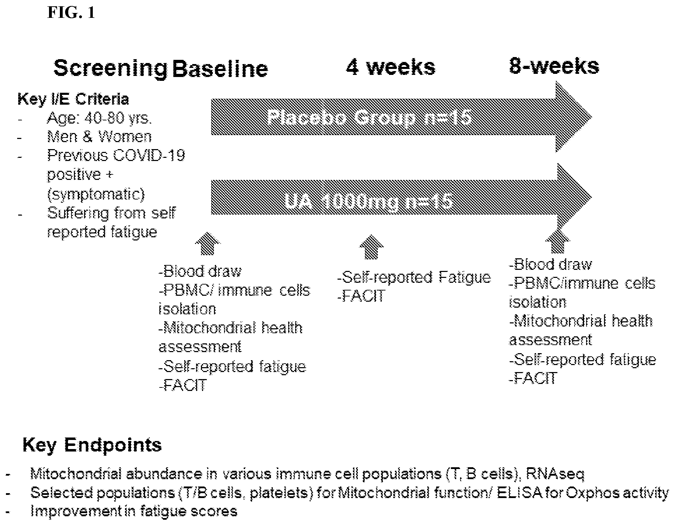

A long COVID-19 human study targeting improvement in mitochondrial function/metabolic fitness of immune cells is performed as described in Fig. 1. Administration of urolithins improves mitochondrial health in immune cells and mitochondrial health in immune cells from post-COVID-19 recovering subjects.

Key endpoints include improvement in subject fatigue scores, which are improved following treatment with a urolithin.

Three patients afflicted with Long COVID were administered 500 mg Mitopure^{™} (Urolithin A). Each patient indicated improvement in symptoms including reduced fatigue and improved muscle function.

### Example 2. Mitochondrial Dysfunction in COVID-19 patients

It has been shown that blood monocytes from patients with COVID-19 pneumonia display signs of mitochondrial dysfunction (Gibellini et al. EMBO Molecular Medicine. 2020, 12, e13001). Monocytes from COVID-19 patients displayed a reduced capacity to maintain maximal respiration when compared to controls. A marked reduction of the spare respiratory capacity was also observed in COVID-19 monocytes, suggesting that the mitochondrial capacity to meet metabolic demands in stressing conditions was compromised (Gibellini *et al.* 2020).

Additionally, it has been shown that SARS-CoV-2 infection results in rapid mitochondrial dysfunction in both CD4 and CD8 T Cells; COVID-19 patients with hyperactivated memory T cells displayed high mitochondrial dysfunction phenotype; and high mitochondrial dysfunction is associated with reduced TEM cell functionality in COVID-19 patients (Mo et al. Front. Immunol. 2022, 12, 799896). SARS-CoV-2-induced mitochondrial dysfunction compromised T-cell functionality, contributing to suppressed T-cell immune responses to viral infection (Mo *et al.* 2022). Also, mitochondrial function and deficient energy supply were observed in PBMCs from COVID-19 patients, which contributed to enhanced inflammatory responses causing disease severity.

Accordingly, improvement of mitochondrial by administration of urolithins is useful to do improve mitochondrial function, thereby treating patients with COVID-19 and subsequent post-COVID diseases or disorders.

### Example 3. Urolithin A Impact Pathways Related to Long COVID19 Development in Human Blood Cells

### Methods

RNA was extracted from frozen human PBMCs and quality of extraction controlled with Agilent Fragment Analyzer System. RNA-seq library was prepared as strand-specific cDNA library, after the following steps: Purification of poly-A containing mRNA molecules; mRNA fragmentation; Random primed cDNA synthesis (strand-specific); Adapter ligation and adapter specific PCR amplification. RNA-seq run was performed with the NovaSeq6000 using S4 flowcells with 2x150bp giving at least 30 million read pairs per sample

The RNA-Seq reads were aligned to the reference human transcriptome using Bowtie generating genome / transcriptome alignments. TopHat identifid the potential exon-exon splice junctions of the initial alignment. Then Cufflinks identified and quantified the transcripts from the preprocessed RNA-Seq alignment assembly. After this, Cuffmerge merged the identified transcript pieces to full length transcripts and annotates the transcripts based on the given annotations. Finally, merged transcripts from two or more samples / conditions were compared, using Cuffdiff to determine the differential expression levels at transcript and gene level including a measure of significance between samples / conditions.

Gene set enrichment analysis (GSEA) was conducted using R package clusterProfiler, v. 3.18.068. Enrichments of Gene Ontology (GO) Biological Process (BP) was investigated. All the genes subjected to the DE analysis and that passed the independent filtering of DEseq2 were ranked by shrunk log2 fold change values and used as input data for the analysis. Ensembl IDs of the genes were converted to Entrez IDs for the analysis using biomaRt, v. 2.46. Minimum and maximum genes et sizes were set to 15 and 500, respectively. In order to adjust for the placebo effect, the GSEA results of Urolithin A at 500 mg were filtered to exclude enriched terms (p-value < 0.05) obtained from the GSEA of the Placebo DEGs. Key pathways were visualised as bar plots ranked by Normalized Enrichment score (NES).

### Discussion

RNA-seq transcriptomic analysis was performed in healthy elderly supplemented with either Urolithin A (UA) at 500 mg/day or Placebo for 28 days. Peripheral blood mononuclear cells (PBMCs) were isolated and collected at baseline and at the end of the study. RNA was extracted from these subjects and used to perform RNA-seq and determine biological pathways modulated specifically by Urolithin A, but not Placebo, comparing end-of treatment to baseline

Fig. 1 shows pathways selectively up- of down-regulated by UA 500 mg after the 28 days administration period compared to baseline.

Among top upregulated pathways, UA induces several gene sets that are associated with better mitochondrial function. Gene sets up-regulated by UA include the Gene Ontology categories: "oxidative phosphorylation" and "mitochondrial respiratory chain complex assembly", that include genes that are associated with improved mitochondrial respiration; and "mitochondrial gene expression", that includes genes involved in mitochondrial ribosome and mitochondrial protein translation components required associated with enhanced mitochondrial biogenesis.

This is relevant in the context of long COVID-19 and ME/CFS, since mitochondrial dysfunction is a well-established feature observed in PBMCs from ME/CFS (Armstrong et al. Metabolomics. 2015, 11, 1626) and reduced mitochondrial function is linked to ME/CFS pathology progression patients, as well as COVID-19 and long COVID-19 (Wood et al. Chronic Diseases and Translational Medicine. 2021, 7, 14).

Among top down-regulated pathways, UA 500 mg/day reduced several gene sets associated with cytokine activation, response to virus infection and inflammation. Gene sets down-regulated by UA include the Gene Ontology categories: "regulation of response to cytokine stimulus" and "neutrophil activation", that include genes involved in activation of immune cells and inflammation, such as the Interleukin 1 Receptor 1 (IL1R1) that mediates the effects of pro-inflammatory cytokine IL1 and the matrix metalloproteinase MMP9 that indicates elevated inflammation.

This is relevant in the context of long COVID-19 and ME/CFS, since COVID-19 severity is associated with an excessive release of pro-inflammatory cytokines (i.e. cytokine storm), by neutrophils and other immune cells. Although cytokines and neutrophils could present a protective role, their excessive and prolonged activation can cause detrimental effects, resulting in pneumonia and/or ARDS (Abraham Crit Care Med. 2003, 31, 4, S195). In COVID19 patients it has been observed high levels of IL1 (Makaremi, S. et al. Inflamm Res. 2022, 71, 923), and MMP9 (Carolina D'Avila-Mesquita, C. et al. Biomedicine & Pharmacotherapy. 2021, 142, 112067) and these high levels are associated with elevated severity of COVID-19 disease.

Therefore, the data in PBMC in subjects supplemented with UA indicate that UA can rescue the same biological processes - reduced mitochondrial function and excessive inflammation - that are associated with ME/CFS, COVID-19 and long COVID-19.

## Claims

1. A composition for use in a method of treating a subject afflicted with long COVID-19, the method comprising administering to the subject a therapeutically effective amount of an urolithin.

2. The composition for use in the method of claim 1, wherein the symptoms comprise fatigue, post-exertional malaise (PEM), problems with memory or concentration, sore throat, headache, muscle or joint pain, dizziness, brain fog, shortness of breath, and/or unrefreshing sleep.

3. The composition for use in the method of claim 1, wherein the urolithin treats a disease or disorder associated with recovery from COVID-19 in the subject, or triggered by COVID-19 in the subject.

4. The composition for use in the method of claim 3, wherein the disease or disorder is myalgic encephalomyelitis/chronic fatigue syndrome.

5. The composition for use in the method of any one of claims 1-4, wherein the long COVID-19 is caused by an infection with a delta variant or an omicron variant of SARS-CoV-2 in the subject.

6. A composition for use in a method of treating a subject afflicted with myalgic encephalomyelitis/chronic fatigue syndrome, the method comprising administering to the subject a therapeutically effective amount of an urolithin.

7. The composition for use in the method of claim 6, wherein the urolithin treats symptoms associated with myalgic encephalomyelitis/chronic fatigue syndrome, optionally wherein the symptoms comprise fatigue, post-exertional malaise (PEM) Problems with memory or concentration, sore throat, headache, muscle or joint pain, dizziness, shortness of breath, or unrefreshing sleep.

8. The composition for use in the method of any one of claims 6-7, wherein the myalgic encephalomyelitis/chronic fatigue syndrome is triggered by COVID-19 or long COVID-19 in the subject.

9. The composition for use in the method of any one of claims 6-8, wherein the subject was previously afflicted with COVID-19 or long COVID-19 prior to the onset of myalgic encephalomyelitis/chronic fatigue syndrome.

10. The composition for use in the method of any one of claims 1-9, wherein the urolithin is urolithin A, urolithin B, urolithin C, or urolithin D, preferably wherein the urolithin is urolithin A.

11. The composition for use in the method of any one of claims 1-10, wherein the subject is concurrently administered a nutrient, wherein the nutrient is vitamin C or zinc.

12. The composition for use in the method of any one of claims 1-11, wherein the subject is concurrently administered a coenzyme, wherein the coenzyme is coenzyme Q10 (CoQ10) or nicotinamide adenine dinucleotide (NAD+).

13. The composition for use in the method of any one of claims 1-12, wherein the subject is concurrently administered a nicotinamide adenine dinucleotide (NAD+) precursor, optionally wherein the nicotinamide adenine dinucleotide (NAD+) precursor is nicotinamide riboside.

14. The composition for use in the method of any one of claims 1-11, wherein the urolithin is present in a pharmaceutical composition further comprising a nutrient, wherein the nutrient is Vitamin C or zinc.

15. The composition for use in the method of any one of claims 1-11, wherein the urolithin is administered in a pharmaceutical composition further comprising a coenzyme or a nicotinamide adenine dinucleotide (NAD+) precursor, wherein the coenzyme is coenzyme Q10 (CoQ10) or nicotinamide adenine dinucleotide (NAD+) and optionally wherein the nicotinamide adenine dinucleotide (NAD+) precursor is nicotinamide riboside.

## Patentansprüche

1. Zusammensetzung zur Verwendung in einem Verfahren zur Behandlung eines an Long-COVID-19 leidenden Subjekts, wobei das Verfahren das Verabreichen einer therapeutisch wirksamen Menge eines Urolithins an das Subjekt umfasst.

2. Die Zusammensetzung zur Verwendung in dem Verfahren nach Anspruch 1, wobei die Symptome Müdigkeit, post-exertionelle Malaise (PEM), Gedächtnis- oder Konzentrationsprobleme, Halsschmerzen, Kopfschmerzen, Muskel- oder Gelenkschmerzen, Schwindel, Brain Fog, Kurzatmigkeit und/oder nicht erholsamen Schlaf umfassen.

3. Die Zusammensetzung zur Verwendung nach Anspruch 1, wobei das Urolithin eine beim Subjekt mit der Erholung von COVID-19 assoziierte oder durch COVID-19 im Subjekt ausgelöste Erkrankung oder Störung behandelt.

4. Die Zusammensetzung zur Verwendung in dem Verfahren nach Anspruch 3, wobei die Erkrankung oder Störung Myalgische Enzephalomyelitis/Chronisches Fatigue-Syndrom ist.

5. Die Zusammensetzung zur Verwendung in dem Verfahren nach einem der Ansprüche 1 bis 4, wobei das Long-COVID-19 durch eine Infektion mit einer Delta-Variante oder einer Omikron-Variante von SARS-CoV-2 in dem Subjekt verursacht worden ist.

6. Zusammensetzung zur Verwendung in einem Verfahren zur Behandlung eines Subjekts, das an Myalgischer Enzephalomyelitis/Chronischem Fatigue-Syndrom leidet, wobei das Verfahren das Verabreichen einer therapeutisch wirksamen Menge eines Urolithins an das Subjekt umfasst.

7. Die Zusammensetzung zur Verwendung in dem Verfahren nach Anspruch 6, wobei das Urolithin mit Myalgischer Enzephalomyelitis/Chronischem Fatigue-Syndrom assoziierte Symptome behandelt, wobei die Symptome optional Müdigkeit, post-exertionelle Malaise (PEM), Gedächtnis- oder Konzentrationsprobleme, Halsschmerzen, Kopfschmerzen, Muskel- oder Gelenkschmerzen, Schwindel, Kurzatmigkeit oder nicht erholsamen Schlaf umfassen.

8. Die Zusammensetzung zur Verwendung in dem Verfahren nach einem der Ansprüche 6 bis 7, wobei die Myalgische Enzephalomyelitis/das Chronische Fatigue Syndrom durch COVID-19 oder Long-COVID-19 in dem Subjekt ausgelöst wird.

9. Die Zusammensetzung zur Verwendung in dem Verfahren nach einem der Ansprüche 6 bis 8, wobei das Subjekt vor dem Einsetzen der Myalgischen Enzephalomyelitis/des Chronischen Fatigue-Syndroms zuvor an COVID-19 oder Long-COVID-19 litt.

10. Die Zusammensetzung zur Verwendung in dem Verfahren nach einem der Ansprüche 1 bis 9, wobei das Urolithin Urolithin A, Urolithin B, Urolithin C oder Urolithin D ist, wobei das Urolithin vorzugsweise Urolithin A ist.

11. Die Zusammensetzung zur Verwendung in dem Verfahren nach einem der Ansprüche 1 bis 10, wobei dem Subjekt gleichzeitig ein Nährstoff verabreicht wird, wobei der Nährstoff Vitamin C oder Zink ist.

12. Die Zusammensetzung zur Verwendung in dem Verfahren nach einem der Ansprüche 1 bis 11, wobei dem Subjekt gleichzeitig ein Coenzym verabreicht wird, wobei das Coenzym Coenzym Q10 (CoQ10) oder Nicotinamidadenindinukleotid (NAD+) ist.

13. Die Zusammensetzung zur Verwendung in dem Verfahren nach einem der Ansprüche 1 bis 12, wobei dem Subjekt gleichzeitig ein Nicotinamidadenindinukleotid (NAD+)-Vorläufer verabreicht wird, wobei der Nicotinamidadenindinukleotid (NAD+)-Vorläufer optional Nicotinamid-Ribosid ist.

14. Die Zusammensetzung zur Verwendung in dem Verfahren nach einem der Ansprüche 1 bis 11, wobei das Urolithin in einer pharmazeutischen Zusammensetzung vorliegt, die weiterhin einen Nährstoff umfasst, wobei der Nährstoff Vitamin C oder Zink ist.

15. Die Zusammensetzung zur Verwendung in dem Verfahren nach einem der Ansprüche 1 bis 11, wobei das Urolithin in einer pharmazeutischen Zusammensetzung verabreicht wird, die weiterhin ein Coenzym oder einen Nicotinamidadenindinukleotid (NAD+)-Vorläufer umfasst, wobei das Coenzym Coenzym Q10 (CoQ10) oder Nicotinamidadenindinukleotid (NAD+) ist und wobei der Nicotinamidadenindinukleotid (NAD+)-Vorläufer optional Nicotinamid-Ribosid ist.

## Revendications

1. Composition destinée à une utilisation dans un procédé de traitement d'un sujet atteint de COVID-19 longue, le procédé comprenant l'administration au sujet d'une quantité thérapeutiquement efficace d'une urolithine.

2. Composition destinée à une utilisation dans le procédé selon la revendication 1, dans laquelle les symptômes comprennent la fatigue, le malaise post-effort (MPE), les problèmes de mémoire ou de concentration, les maux de gorge, les maux de tête, les douleurs musculaires ou articulaires, les vertiges, le brouillard cérébral, l'essoufflement et/ou un sommeil non réparateur.

3. Composition destinée à une utilisation dans le procédé selon la revendication 1, dans laquelle l'urolithine traite une maladie ou un trouble associé à la guérison de la COVID-19 chez le sujet, ou déclenché par la COVID-19 chez le sujet.

4. Composition destinée à une utilisation dans le procédé selon la revendication 3, dans laquelle la maladie ou le trouble est l'encéphalomyélite myalgique/le syndrome de fatigue chronique.

5. Composition destinée à une utilisation dans le procédé selon l'une quelconque des revendications 1 à 4, dans laquelle la COVID-19 longue est causée par une infection par une variante delta ou une variante omicron du SARS-CoV-2 chez le sujet.

6. Composition destinée à une utilisation dans un procédé de traitement d'un sujet atteint d'encéphalomyélite myalgique/syndrome de fatigue chronique, le procédé comprenant l'administration au sujet d'une quantité thérapeutiquement efficace d'une urolithine.

7. Composition destinée à une utilisation dans le procédé selon la revendication 6, dans laquelle l'urolithine traite les symptômes associés à l'encéphalomyélite myalgique/au syndrome de fatigue chronique, facultativement dans laquelle les symptômes comprennent la fatigue, le malaise post-effort (MPE), les problèmes de mémoire ou de concentration, les maux de gorge, les maux de tête, les douleurs musculaires ou articulaires, les vertiges, l'essoufflement ou un sommeil non réparateur.

8. Composition destinée à une utilisation dans le procédé selon l'une quelconque des revendications 6 et 7, dans laquelle l'encéphalomyélite myalgique/le syndrome de fatigue chronique est déclenché par la COVID-19 ou la COVID-19 longue chez le sujet.

9. Composition destinée à une utilisation dans le procédé selon l'une quelconque des revendications 6 à 8, dans laquelle le sujet était déjà atteint de COVID-19 ou de COVID-19 longue avant l'apparition de l'encéphalomyélite myalgique/du syndrome de fatigue chronique.

10. Composition destinée à une utilisation dans le procédé selon l'une quelconque des revendications 1 à 9, dans laquelle l'urolithine est l'urolithine A, l'urolithine B, l'urolithine C ou l'urolithine D, de préférence dans laquelle l'urolithine est l'urolithine A.

11. Composition destinée à une utilisation dans le procédé selon l'une quelconque des revendications 1 à 10, dans laquelle le sujet reçoit simultanément un nutriment, dans laquelle le nutriment est de la vitamine C ou du zinc.

12. Composition destinée à une utilisation dans le procédé selon l'une quelconque des revendications 1 à 11, dans laquelle le sujet reçoit simultanément une coenzyme, dans laquelle la coenzyme est la coenzyme Q10 (CoQ10) ou le nicotinamide adénine dinucléotide (NAD+).

13. Composition destinée à une utilisation dans le procédé selon l'une quelconque des revendications 1 à 12, dans laquelle le sujet reçoit simultanément un précurseur de nicotinamide adénine dinucléotide (NAD+), facultativement dans lequel le précurseur de nicotinamide adénine dinucléotide (NAD+) est le riboside de nicotinamide.

14. Composition destinée à une utilisation dans le procédé selon l'une quelconque des revendications 1 à 11, dans laquelle l'urolithine est présente dans une composition pharmaceutique comprenant en outre un nutriment, dans laquelle le nutriment est de la vitamine C ou du zinc.

15. Composition destinée à une utilisation dans le procédé selon l'une quelconque des revendications 1 à 11, dans laquelle l'urolithine est administrée dans une composition pharmaceutique comprenant en outre une coenzyme ou un précurseur de nicotinamide adénine dinucléotide (NAD+), dans laquelle la coenzyme est la coenzyme Q10 (CoQ10) ou le nicotinamide adénine dinucléotide (NAD+) et, facultativement, dans laquelle le précurseur de nicotinamide adénine dinucléotide (NAD+) est le riboside de nicotinamide.
